# FASCICULE DE BREVET EUROPEEN

(11) **EP 2 734 514 B1**
(45) Date de publication et mention de la délivrance du brevet: **08.07.2015**
(21) Numéro de dépôt: 12748512.6
(22) Date de dépôt: 16.07.2012
(51) Int. Cl.: C07D 333/34, G01N 1/00

(54) **UTILISATION D'UN COMPOSÉ DÉRIVÉ DU THIOPHÈNE COMME MATRICE DANS LA DÉTECTION SPÉCIFIQUE, L'IDENTIFICATION ET/OU LA QUANTIFICATION D'ALCALOÏDES PAR SPECTROMÉTRIE DE MASSE MALDI-TOF**
VERWENDUNG EINER THIOPHENVERBINDUNG ALS MATRIX BEI DER SPEZIFISCHEN DETEKTION, IDENTIFIZIERUNG UND/ODER QUANTIFIZIERUNG VON ALKALOIDEN MITTELS MALDI-TOF-MASSENSPEKTROMETRIE
USE OF A THIOPHENE COMPOUND AS A MATRIX IN THE SPECIFIC DETECTION, IDENTIFICATION AND/OR QUANTIFICATION OF ALKALOIDS BY MALDI-TOF MASS SPECTROMETRY

(30) Priorité: 18.07.2011 FR 1156523
(43) Date de publication de la demande: 28.05.2014
(73) Titulaire: UNIVERSITE D'ANGERS, 49035 Angers (FR)
(72) Inventeur: DIAS, Marylène, F-49460 Soulaire et Bourg (FR); LEVILLAIN, Eric, F-49460 Soulaire et Bourg (FR); RICHOMME, Pascal, F-49000 Angers (FR); SCHINKOVITZ, Andreas, F-49100 Angers (FR); SERAPHIN, Denis, F-49320 Saint-Saturnin-sur-Loire (FR)
(74) Mandataire: Novagraaf Technologies
(86) Numéro de dépôt international: PCT/FR2012/051687
(87) Numéro de publication internationale: WO 2013/011234

(56) Documents cités:
- JP-A- 5 320 158
- SHIN-ICHI TANIGUCHI ET AL: "Electron transport in networks of gold nanoparticles connected by oligothiophene molecular wires", JOURNAL OF MATERIALS CHEMISTRY, THE ROYAL SOCIETY OF CHEMISTRY, CAMBRIDGE, GB, vol. 16, no. 34, 14 septembre 2006 (2006-09-14), pages 3459-3465, XP002623088, ISSN: 0959-9428, DOI: 10.1039/B604732G [extrait le 2006-07-28]
- L. SANGUINET ET AL: "Desorption/ionization on self-assembled monolayer surfaces (DIAMS)", JOURNAL OF MASS SPECTROMETRY, vol. 41, no. 6, 1 juin 2006 (2006-06-01), pages 830-833, XP055011319, ISSN: 1076-5174, DOI: 10.1002/jms.1036
- TRACY DONOVAN MCCARLEY ET AL: "Electron-Transfer Ionization in Matrix-Assisted Laser Desorption/Ionization Mass Spectrometry", ANALYTICAL CHEMISTRY, vol. 70, no. 20, 1 octobre 1998 (1998-10-01), pages 4376-4379, XP055011321, ISSN: 0003-2700, DOI: 10.1021/ac980527i
- ANDREAS WOLDEGIORGIS ET AL: "Polymer-assisted laser desorption/ionization analysis of small molecular weight compounds", RAPID COMMUNICATIONS IN MASS SPECTROMETRY, vol. 18, no. 8, 30 avril 2004 (2004-04-30) , pages 841-852, XP055011496, ISSN: 0951-4198, DOI: 10.1002/rcm.1412
- MATTHIEU BOUNICHOU ET AL: "Self-assembled monolayer-assisted mass spectrometry", JOURNAL OF MATERIALS CHEMISTRY, vol. 19, no. 43, 1 janvier 2009 (2009-01-01), page 8032, XP055011472, ISSN: 0959-9428, DOI: 10.1039/b904175n
- COHEN L H ET AL: "Small molecule analysis by MALDI mass spectrometry", ANALYTICAL AND BIOANALYTICAL CHEMISTRY, SPRINGER, DE, vol. 373, no. 7, 1 août 2002 (2002-08-01), pages 571-586, XP007913578, ISSN: 1618-2642, DOI: 10.1007/S00216-002-1321-Z [extrait le 2004-02-19]
- ANDREAS SCHINKOVITZ ET AL: "Selective detection of alkaloids in MALDI-TOF: the introduction of a novel matrix molecule", ANALYTICAL AND BIOANALYTICAL CHEMISTRY, SPRINGER, BERLIN, DE, vol. 403, no. 6, 20 avril 2012 (2012-04-20), pages 1697-1705, XP035057321, ISSN: 1618-2650, DOI: 10.1007/S00216-012-5958-Y

## Description

### Domaine technique

La présente invention concerne (i) une méthode d'analyse de molécules de faible masse pouvant être < 800 Da, en particulier des alcaloides, ladite méthode étant généralement désignée sous le nom de MALDI-TOF-MS qui est un acronyme pour méthode d'analyse par spectrométrie de masse à temps de vol à désorption/ionisation sous laser assistée par matrice et (ii) une nouvelle utilisation d'un composé en tant que matrice dans une telle méthode d'analyse.

Dans cette méthode, des impulsions de laser sont concentrées sur un échantillon plat contenant les molécules à analyser, incorporées dans une matrice. La matrice absorbe la majeure partie de l'énergie du laser et, consécutivement, par le transfert de protons, ionise et vaporise les molécules à analyser.

Dans la description ci-dessous, les références entre crochets ([ ]) renvoient à la liste des références présentée à la fin du texte.

### Etat de la technique

Un instrument de type MALDI-TOF-MS est un spectromètre de masse couplant une source d'ionisation laser assistée par une matrice, abrégé MALDI selon l'acronyme anglais « *Matrix-Assisted Laser Desorptionllonisation* » avec un analyseur à temps de vol, abrégé TOF selon l'acronyme anglais «*Time*-*Of-Flight* ». Il permet de réaliser des méthodes d'analyse définies ci-après.

La méthode d'analyse MALDI-TOF-MS est connue pour trouver une utilisation accrue dans la détermination de la masse de macromolécules non-volatiles.

En effet, la désorption-ionisation laser assistée par matrice est une technique d'ionisation douce utilisée en spectrométrie de masse, permettant l'analyse de biomolécules, telles que des biopolymères comme les protéines, les peptides et les hydrates de carbone, et de molécules organiques comme les polymères, les dendrimères et autres macromolécules, qui tendent à devenir fragiles et à se fragmenter lorsqu'elles sont ionisées par des méthodes plus conventionnelles. La technique MALDI a ainsi permis d'étendre le champ d'application de la spectrométrie de masse. Traditionnellement, la méthode MALDI-TOF-MS n'est pas appropriée pour l'analyse de molécules de faibles masses, en particulier ayant une m/z < 800.

L'ionisation est provoquée par un faisceau laser, normalement un laser à l'azote. Le laser est dirigé sur les cristaux formant la matrice du spot MALDI qui absorbe l'énergie laser, la matrice étant utilisée afin de protéger les molécules à analyser de la destruction par un faisceau direct et de faciliter la vaporisation et l'ionisation.

La matrice est ionisée tout d'abord par ce phénomène et transfère ensuite une partie de sa charge aux molécules à analyser, les ionisant tout en les protégeant d'une énergie disruptive du laser. Le processus de mise en équilibre des ions prend approximativement 100 ns ou moins après que la plupart des ions a quitté la surface avec une certaine vitesse moyenne. Il en résulte une dispersion dans le temps de départ des ions. Pour compenser cette dispersion et ainsi améliorer la résolution en masse, l'extraction des ions de la source vers l'analyseur est retardée de quelques centaines de nanosecondes, voire de quelques microsecondes. Cette technique est connue sous la terminologie d'« extraction retardée » pour la désorption/ionisation.

La spectrométrie de masse à temps de vol abrégé TOF-MS selon l'acronyme anglais *« Time of Flight Mass Spectrometry* » est une méthode d'analyse de spectrométrie de masse dans laquelle les ions sont accélérés par un champ électrique de valeur connue. Il résulte de cette accélération que les ions de même charge électrique acquièrent la même énergie cinétique. La vitesse des ions, par contre dépend du rapport masse/charge. On mesure le temps mis par une particule chargée pour atteindre un détecteur situé à une distance connue. Ce temps dépendra du rapport masse/charge de la particule considérée. Ce sont les particules les plus lourdes qui seront accélérées aux vitesses les plus basses. La détermination du rapport masse/charge découle de ce temps de vol et de la connaissance des autres paramètres expérimentaux comme la position du détecteur et la tension d'accélération.

L'une des caractéristiques importantes d'une spectrométrie de masse est la finesse des pics, mesurée par la résolution du spectromètre de masse. La résolution est définie comme étant le rapport de la masse m du pic sur la largeur à mi-hauteur Δm. Plus la résolution est élevée, plus les pics sont fins. Il est alors possible de visualiser deux molécules de masses proches.

Les instruments MALDI-TOF-MS peuvent être équipés avec un réflectron (miroir électrostatique ou « miroir à ions ») qui dévie les ions avec un champ électrique, doublant approximativement la longueur du chemin de vol de l'ion et augmentant la résolution de l'instrument. Un spectromètre de masse MALDI-TOF peut atteindre des résolutions de 5000 en mode linéaire (sans réflectron) et de 20000 avec réflectron.

Historiquement, les analyseurs par temps de vol, qui nécessitent une source d'ionisation pulsée, étaient couplés uniquement avec les sources MALDI. Actuellement, les sources MALDI peuvent être couplées à d'autres types d'analyseurs (par exemple un analyseur FT-ICR) et l'analyseur par temps de vol à d'autres sources (par exemple avec une source électrospray dans un instrument ESI-QTOF).

Des explications supplémentaires liées à la méthode de MALDI-TOF-MS peuvent être trouvées dans les articles suivants: U.S. No. 6.104.028 ; « The Scientist » 13 [12] : 18, le 7 juin 1999 ; et « Biophotonics International», juin 2001, 42-47.

En outre, la nature de la matrice s'avère très importante dans la méthode d'analyse MALDI-TOF-MS. La matrice est généralement une petite molécule organique qui peut absorber le rayonnement laser intense empêchant de ce fait la décomposition des molécules à analyser mais qui peut aussi doucement transférer l'énergie aux molécules à analyser et favoriser ainsi leur ionisation.

La nature des molécules pour fabriquer une matrice efficace est déterminée par tâtonnement, mais reste basée sur des considérations spécifiques de « profils moléculaires ». Lesdites molécules :
- doivent être de faible masse moléculaire afin de faciliter la vaporisation, mais suffisamment grosses avec une pression de vapeur assez grande afin de ne pas s'évaporer durant la préparation de l'échantillon ou lors de son introduction dans le spectromètre;
- doivent être acides, ou au moins agir comme source de proton(s) afin de favoriser l'ionisation des molécules à analyser ;
- doivent avoir une forte absorption dans l'ultraviolet, leur permettant d'absorber efficacement et rapidement l'irradiation laser ; et
- doivent être fonctionnalisées avec des groupes polaires, pour une utilisation en solutions aqueuses.

Dans un premier temps, une matrice en solution dans un solvant ou dans l'eau est mélangée avec l'échantillon à analyser. Un solvant organique permet aux molécules hydrophobes de se dissoudre dans la solution, alors que l'eau permet aux molécules hydrophiles de faire de même. Une solution avec l'une de ces molécules est réalisée, parfois dans un mélange d'eau ultra-pure et de solvant organique, habituellement de l'acétonitrile, abrégé ACN, ou de l'éthanol, abrégé EtOH. L'acide trifluoroacétique, abrégé TFA, peut parfois être ajouté. Un bon exemple de solution-matrice est, par exemple, un mélange de 20 mg/mL d'acide sinapique dans un mélange ACN/eau/TFA (50/50/0,1 en volume).

La solution ainsi obtenue est ensuite, dans un second temps, déposée sur une coupelle MALDI, habituellement en métal conçue pour cet usage. Ledit dépôt est réalisé selon différentes techniques de dépôt, manuelles ou automatisées, qui prennent aussi toute leur importance dans la qualité des spectres obtenus. Le solvant se vaporise à température ambiante et sous pression réduite, laissant la matrice recristallisée, mais avec maintenant des molécules à analyser réparties dans tout le cristal. La matrice et le ou les composé(s) à analyser sont dits alors co-*cristallisés* dans un spot MALDI.

Une liste d'exemples de composés utilisables comme matrice en fonction des molécules à analyser est répertoriée dans le **tableau 1** ci-dessous. Une matrice donnée peut s'avérer spécifique à certaines molécules que l'on cherche à détecter, identifier ou quantifier.

**Tableau 1**

| **Composé** | **Abréviation** | **Utilisation** |
|---|---|---|
| 9-nitroanthracène | 9-NA | Fullerènes |
| Acide alpha-cyano-4-hydroxycinnamique | HCCA | Peptides de moins de 10 kDa Hydrates de carbone |
| Acide sinapique | SA | Protéines de plus de 10 kDa Fullerenes |
| Acide 2-(4-hydroxyphénylazo)benzoïque | HABA | Protéines de plus de 10 kDa |
| 2,4,6-Trihydroxyacétophénone | THAP | Oligonucléotides de moins de 3,5 kDa Hydrates de carbone acide |
| Acide 3-hydroxypicolinique | HPA | Oligonucléotides de plus de 3,5kDa |
| Acide anthranilique | - | Oligonucléotides de plus de 3,5 kDa |
| Acide nicotinique | - | Oligonucléotides de plus de 3,5 kDa |
| Acide trans-3-indoleacrylique | IAA | Polymères synthétiques non polaires |
| Dithranol | DIT | Polymères synthétiques non polaires Lipides |
| Acide 2,5-dihydroxybenzoïque | DHB | Polymères synthétiques polaires Molécules organiques Hydrates de carbone |
| 1-Isoquinolinol | - | Oligosaccharides |
| Acide picolinique | - | Oligonucléotides |
| 2,5-dihydroxyacétophénone | DHAP | Protéines de plus de 10 kDa |

La matrice consiste en des molécules cristallisées, qui sont, en général, choisies parmi les trois suivantes : l'acide 3,5-diméthoxy-4-hydroxycinnamique (acide sinapique/acide sinapinique), l'acide alpha-cyano-4-hydroxycinnamique (alpha-cyano ou alpha-matrice) et l'acide 2,5-dihydroxybenzoique abrégé DHB.

En MALDI-TOF-MS, si la matrice joue un rôle essentiel lors du processus de désorption/ionisation, elle représente également un facteur limitant de la technique dans la mesure où elle génère très souvent et en quantités importantes des ions dans la gamme des bas rapports de masse sur charge (m/z). Ce phénomène rend ainsi la caractérisation des petites molécules, typiquement < 800 Da, très délicate.

Il existe donc un réel besoin d'une matrice palliant les inconvénients et obstacles de l'art antérieur, en particulier d'un procédé permettant d'analyser de petites molécules < 800 Da, telles que par exemple les alcaloïdes, en particulier de les caractériser et/ou les quantifier à partir d'un mélange complexe.

Le document CN 101644694 A décrit la détection directe par MALDI-TOF-MS d'alcaloides (l'aconitine, la berbérine, la strychnine) dans des extraits bruts obtenus à partir de diverses plantes de la Pharmacopée traditionnelle chinoise : *Radix aconiti, Rhizoma typhoni, Cortex phellodendri.* La matrice DHB utilisée est classique et non spécifique aux alcaloïdes. De plus, le procédé d'analyse divulgué dans ce document ne permet pas la quantification des molécules détectées.

Le document « Journal of Mass Spectrometry », 2007 ; 42 :58-69 décrit une méthode de détermination directe de profils d'alcaloïdes dans des tissus végétaux tels que des herbes chinoises, utilisant la spectrométrie de masse MALDI-TOF. Les matrices d'acide alpha-cyano-4-hydroxycinnamique, abrégé CHCA, et d'acide 2,5-dihydroxybenzoique, abrégé DHB, sont citées.

Le document « Analytica Chimica Acta », 649 (2009), 230-235 concerne une matrice de 7-mercapto-4-méthylcoumarine qui est appropriée pour l'analyse par MALDI de composés de petit poids moléculaire, tels que des alcaloïdes carcinogéniques, en particulier les alcaloides arécoline et arécaidine. Le document « Analytical Chemistry », 1998, 70, 4376-4379, concerne une matrice de terthiophène pour l'analyse par MALDI de métallocènes.

Enfin, le document « Journal of Mass Spectrometry », 2006, 41 : 830-833, décrit l'utilisation d'un dérivé du thiophène dans une technique de désorption/ionisation laser sans matrice qui utilisent des surfaces modifiées.

La présente invention repose donc sur la découverte de nouvelles molécules et sur leur utilisation comme matrice de MALDI-TOF ou l'utilisation d'une molécule connue par ailleurs, le 3-(5-(5-(méthylthio)thiophén-2-yl)thiophén-2-ylthio)propanenitrile abrégé MT3P, mais inconnue dans l'art antérieur pour un usage en MALDI-TOF-MS, ces molécules ayant l'avantage de générer très peu d'ions "parasites" et pouvant conduire à l'ionisation de petites molécules, avantageusement de masse moléculaire, abrégé MM, < 800 Da, plus particulièrement des molécules d'alcaloïdes. Ladite matrice présentant une très grande sensibilité et/ou sélectivité, la quantification desdites petites molécules s'en trouve d'autant améliorée par rapport aux cas dans lesquels les matrices de l'art antérieur sont utilisées. Il est donc possible avec la matrice selon l'invention de caractériser et quantifier, y compris à partir de mélanges complexes tels que des extraits végétaux bruts, des fluides biologiques..., des molécules de MM < 800 Da de très grand intérêt pharmacologique.

L'invention a donc pour objet l'utilisation du composé de formule (I) : 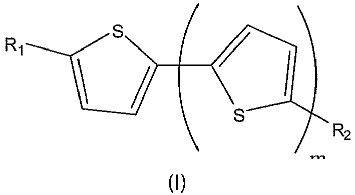 dans laquelle **m** est égal à 1, le groupement **R1** est choisi parmi les groupements -S(CH₂)ₙ-Y avec **n** un nombre entier choisi parmi 2 et 3 et **Y** un groupe fonctionnel choisi parmi-CN, -CO₂R3 et -OH, avec **R3** un atome d'hydrogène ou un groupement méthyle, dans laquelle le groupement **R2** est choisi parmi l'atome d'hydrogène et le groupement -SCH₃, comme matrice dans un dispositif de spectrométrie de masse à désorption/ionisation sous laser, assistée par une matrice ou MALDI.

L'invention a également pour objet un composé de formule (I) : 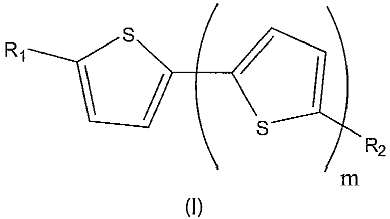 dans laquelle **m** est égal à 1, le groupement **R1** est choisi parmi les groupements -S(CH₂)ₙ-Y avec **n** un nombre entier choisi parmi 2 et 3 et **Y** un groupe fonctionnel choisi parmi-CN, -CO₂R3 et -OH, avec **R3** un atome d'hydrogène ou un groupement méthyle, et dans laquelle le groupement **R₂** est le groupement -SCH₃, ou dans laquelle m = 1, R1 est un groupement -S(CH₂)₂CN et R2 est un atome d'hydrogène ; à l'exclusion du 3-(5-(5-(méthylthio)thiophén-2-yl)thiophén-2-ylthio)propanenitrile abrégé MT3P.

De plus, l'invention couvre aussi l'utilisation d'un composé tel que défini précédemment ou de 3-(5-(5-(méthylthio)thiophén-2-yl)thiophén-2-ylthio)propanenitrile pour fabriquer une matrice destinée à être utilisée dans un dispositif de spectrométrie de masse à désorption/ionisation sous laser assistée par matrice ou MALDI.

En outre, l'invention concerne un procédé de fabrication d'une matrice destinée à être utilisée dans un dispositif de spectrométrie de masse à désorption/ionisation sous laser assistée par matrice ou MALDI consistant à cristalliser un composé tel que défini précédemment ou le composé 3-(5-(5-(méthylthio)thiophén-2-yl)thiophén-2-ylthio)propanenitrile.

Enfin, l'invention est également relative à un procédé de caractérisation et/ou de quantification de molécules, ayant avantageusement une masse < 800 Da, présentes dans un échantillon par spectrométrie de masse à temps de vol à désorption/ionisation sous laser assistée par matrice comprenant une étape de :
➢ Fabrication d'une matrice avec un composé tel que défini précédemment ou avec un composé de 3-(5-(5-(méthylthio)thiophén-2-yl)thiophén-2-ylthio)propanenitrile ;
➢ Mélange dudit échantillon avec ladite matrice, éventuellement en présence d'un solvant organique ou d'eau ;
➢ Vaporisation dudit solvant ou de l'eau, le cas échéant;
➢ Co-cristallisation de ladite matrice et desdites molécules et formation d'un cristal de matrice comprenant lesdites molécules réparties dans tout ledit cristal ;
➢ Ionisation par un faisceau laser de ladite matrice co-cristallisée avec l'échantillon, obtenue précédemment ;
➢ Etablissement d'un spectrogramme ;

Ledit échantillon pouvant être constitué de phanères ou de leurs extraits, ou d'un liquide biologique choisi parmi le sang, le plasma, les liquides des muqueuses génitales, les liquides de la peau, les liquides d'épanchement et des cavités closes, les liquides du système digestif et urinaire.

### Brève description des figures

La **figure 1** représente le spectre de Masse (LDI) du MT3P (I) enregistré en mode linéaire (Biflex III Bruker) pour une intensité du laser de 10 %. Les ions majoritaires correspondent à l'ion pseudomoléculaire [M+H]⁺ (m/z 297.018) d'une part et au fragment [(M+ H)-C₃H₄N]⁺(m/z 243.009) d'autre part.
La **figure 2** représente le spectre MALDI-TOF d'un extrait brut (EtOH/H₃O⁺) de *Senecio vulgaris* (Asteraceae) avec le MT3P (I) pour matrice.
La **figure 3** représente l'abaque I = f(C) avec I : intensité de l'ion pseudomoléculaire et C : concentration nanomolaire de l'analyte, tracée à partir de spectres MALDI-TOF, avec le MT3P pour matrice, de la codéine (**2**).
La **figure 4** représente l'abaque I = f(C) avec 1 : intensité de l'ion pseudomoléculaire et C : concentration nanomolaire de l'analyte, tracée à partir de spectres MALDI-TOF, avec le MT3P pour matrice, de l'hyoscyamine (**3**).

### Description de l'invention

Dans le cadre de l'invention, on entend par :
- « extrait organique brut», tout extrait obtenu à partir de matières fraîches ou sèches issues d'êtres vivants tels que, par exemple, Archées, Bactéries, Protistes, Champignons, Plantes, Animaux et n'ayant fait l'objet d'aucune étape ultérieure de purification. On distingue, en particulier, les extraits végétaux bruts.
- « liquide biologique », tous les liquides issus des êtres vivants tels que par exemple les êtres vivants d'origine humaine ou animale. On peut citer, par exemple, comme liquide biologique le sang, le plasma, l'urine, le sperme...
- « solvant organique », tout composé hydrocarboné utilisé seul ou en association pour solubiliser un ou plusieurs produits.
- « alcaloïdes », des molécules organiques hétérocycliques azotées basiques pouvant avoir une activité pharmacologique. Habituellement, les alcaloïdes sont des dérivés des acides aminés. On trouve des alcaloïdes, en tant que métabolites secondaires, principalement chez les végétaux, les champignons et quelques groupes animaux peu nombreux. Outre les alcaloïdes d'origine naturelle, il existe également des alcaloïdes synthétiques fabriqués par synthèse telle que la chloroquine, la chloroquinine ou par hémisynthèse. Il existe par exemple un type d'alcaloïdes contenant deux atomes d'azote dans le noyau aromatique et qui n'est pas d'origine naturelle, c'est le groupe des pyrazoles.

Bien que beaucoup d'alcaloïdes soient toxiques (comme la strychnine ou l'aconitine), certains sont employés dans la médecine pour, par exemple, leurs propriétés analgésiques (comme la morphine, la codéine), dans le cadre de protocoles de sédation (anesthésie) souvent accompagnés d'hypnotiques, ou comme agent antipaludéen (quinine, chloroquinine) ou agent anticancéreux ( vinblastine, vincristine).
- « groupement alkyle » un groupement hydrocarboné aliphatique, linéaire ou ramifié, comprenant par exemple un atome de carbone (abrégé C1) à six atomes de carbone (abrégé C6).
- « groupement cycloalkyle », un alkyle cyclique, c'est-à-dire un groupement hydrocarboné aliphatique et cyclique comprenant par exemple de trois atomes de carbone (abrégé C3) à six atomes de carbone (abrégé C6).
- « groupement aryle » un groupement aromatique pouvant comprendre au moins un hétéroatome.

L'invention a donc pour objet l'utilisation du composé de formule (I) : 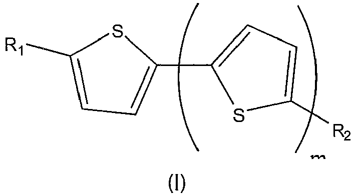 dans laquelle **m** est égal à 1, le groupement **R1** est choisi parmi les groupements -S(CH₂)ₙ-Y avec **n** un nombre entier choisi parmi 2 et 3 et **Y** un groupe fonctionnel choisi parmi-CN, -CO₂R3 et -OH, avec **R3** un atome d'hydrogène ou le groupement -SCH₃, et dans laquelle le groupement **R2** est choisi parmi l'atome d'hydrogène, et le groupement -SCH₃, comme matrice dans un dispositif de spectrométrie de masse à désorption/ionisation sous laser, assistée par une matrice ou MALDI. Le dispositif MALDI peut être couplé à un analyseur à temps de vol.

Avantageusement, il s'agit de composés de formule (I) dans laquelle :
- m = 1, R1 est un groupement -S(CH₂)₂CN et R2 un groupe -SCH₃ ;
- m=1, R1 est un groupement -S(CH₂)₂CO₂CH₃ et R2 est un groupe - SCH₃ ;
- m = 1, R1 est un groupement -S(CH₂)₃CN et R2 est un groupe -SCH₃ ;
- m=1, R1 est un groupement -S(CH₂)₂CN et R2 est un atome d'hydrogène ;
- m = 1, R1 est un groupement -S(CH₂)₂CO₂H et R2 est un groupe -SCH₃ ; ou
- m = 1, R1 est un groupement -S(CH₂)₂-CH₂OH et R2 est un groupe - SCH₃.

Selon un mode de réalisation particulièrement préféré, le composé de formule (I) est le 3-(5-(5-(méthylthio)thiophén-2-yl)thiophén-2-ylthio)propanenitrile ou MT3P.

Une telle utilisation est particulièrement avantageuse pour analyser qualitativement et quantitativement une molécule de masse < 800 Da ou un mélange de molécules ou un échantillon d'extrait organique brut ou de liquide biologique, ledit mélange ou ledit échantillon comprenant au moins une molécule de masse < 800 Da, ladite molécule de masse < 800 Da pouvant être choisie parmi les alcaloïdes tels que définis ci-après.

Les alcaloïdes sont catégorisés en fonction de leur structure chimique. On distingue le :
- Groupe des Azolidines (pyrrolidines) : Aniracetam, Anisomycin, CX614, Dextromoramide, Diphenyl prolinol, acide domoique, Histapyrrodine, acide kainique, Methdilazine, Oxaceprol, Prolintane, Pyrrobutamine, hygrine, cuscohygrine.
- Groupe des Azines : Pipéridine, Conicine, Trigonelline, Arécaidine, Guvacine, Pilocarpine, Cytisine, Nicotine, Spartéine, Pelletierine.
- Groupe des Tropanes : Atropine, Hyoscyamine, Cocaïne, Ecgonine, Scopolamine.
- Groupe des Quinoléines : Acridine, Acide bicinchoninique, Broxyquinoline, Chlorquinaldol, Cinchophen, Clioquinol, Dequalinium, Dihydroquinine, Dihydroquinidine, Hydroxychloroquine, 8-Hydroxyquinoline, Iodoquinol, Acide Kynurenique, Mefloquine, Nitroxoline, Oxycinchophen, Primaquine, Quinine, Quinidine, TSQ, Topotecan, acide xanthurénique, Strychnine, Brucine, Veratrine, Cevadine, Echinopsine ;
   ∘ Aminoquinoléines : Chloroquine, Hydroxychloroquine, Primaquine ;
   ∘ 8-Aminoquinoléines :Tafenoquine, Rhodoquine, Pamaquine .
- Groupe des Isoquinolines : Dimethisoquine, Quinapril, Quinapirilat, Debrisoquine, 2,2'-Hexadecaméthylènediisoquinolinium dichlorure, N-laurylisoquinolinium bromure, Narcéine, Hydrastine, Berbérine;
   ∘ Les alcaloïdes de l'opium :
      Naturels : Morphine, Codéine, Thébame, Papavérine, Narcotine, Noscapine;
      Semi-synthétiques : Hydromorphone, Hydrocodone, Héroine ;
      Synthétiques : Fentanyl, Pethidine, Methadone, Propoxyphène.
- Groupe des Indoles :
   ∘ Ergolines : Les alcaloides de l'ergot de seigle (Ergométrine, Ergotamine, Ergosine, Ergovaline, Ergokryptine, Ergocornine, Ergocristine, Acide lysergique, etc.), Ergine, LSD ...
   ∘ Bêta-carbolines : Harmine, Yohimbine, Réserpine, Emétine.
- Groupe des Terpénoides :
   ∘ Les alcaloïdes de l'aconit napel : Aconitine ;
   ∘ Solanidine, Solasodine, Batrachotoxine, Delphinine ;
   ∘ Stéroides : Solanine, Samandarin.
- Groupe des Bétaïnes (composés d'ammonium quaternaire, ce ne sont pas des alcaloïdes au sens propre, même si régulièrement classés comme tels) : Muscarine, Choline, Neurine.
- Groupes des Pyrazoles.

Ladite molécule de masse < 800 Da peut être choisie parmi 12-Déméthylthalrugosidine, Aconitine, Atropine, Berbérine, Boldine, Cholchicine, Clavuline, Codéine, Émétine, Fumaritine, Harmine, L-Hyoscyamine, Limogine, Morphine, Nicotine, Pilocarpine, Quinidine, Sénécionine, Spartéine, Strychnine, Stylopine, Thalfoétidine, Thalibérine, Thaliglucinone et Yohimbine.

Le ou les molécules de masse < 800 Da et/ou le ou les molécules d'alcaloïdes définis ci-dessus peuvent être ainsi détectées et quantifiées dans des échantillons de liquides biologiques ou d'extrait organiques bruts, en particulier d'extrait végétaux bruts, sans nécessiter d'étapes de purification ou de séparation préalables par spectrométrie de masse sous laser assistée par matrice.

Selon un mode de réalisation, l'échantillon est choisi parmi des extraits organiques bruts, ou toute fraction obtenue à partir desdits extraits organiques bruts, et des liquides ou des échantillons biologiques, d'origine humaine ou animale. Parmi les échantillons biologiques, on peut par exemple citer les phanères tels que les ongles ou les cheveux.

Le liquide biologique peut être choisi parmi le sang, le plasma, les liquides des muqueuses génitales, le liquide séminale, le sperme, la glaire cervicale, les liquides de la peau telle que la sueur, les liquides d'épanchement et des cavités closes, les liquides du système digestif et urinaire.

L'invention a également pour objet un composé de formule (I) : 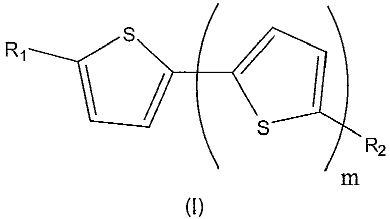 dans laquelle **m** est égal à 1, le groupement **R1** est choisi parmi les groupements -S(CH₂)ₙ-Y avec **n** un nombre entier choisi parmi 2 et 3 et **Y** un groupe fonctionnel choisi parmi-CN, -CO₂R3 et -OH, avec **R3** un atome d'hydrogène ou un groupement méthyle, et dans laquelle le groupement **R2** est le groupement -SCH₃, ou dans laquelle m = 1, R1 est un groupement -S(CH₂)₂CN et R2 est un atome d'hydrogène ;
à l'exclusion du 3-(5-(5-(méthylthio)thiophén-2-yl)thiophén-2-ylthio)propanenitrile.

Selon un mode de réalisation, il s'agit des composés, où :
- m = 1, R1 est un groupement -S(CH₂)₂CO₂CH₃ et R2 est un groupe-SCH₃ ;
- m = 1, R1 est un groupement -S(CH₂)₃CN et R2 est un groupe -SCH₃ ;
- m = 1, R1 est un groupement -S(CH₂)₂CO₂H et R2 est un groupe -SCH₃ ; ou
- m=1, R1 est un groupement -S(CH₂)₂-CH₂OH et R2 est un groupe-SCH₃.

L'invention concerne aussi un procédé de fabrication d'une matrice destinée à être utilisée dans un dispositif de spectrométrie de masse à désorption/ionisation sous laser assistée par matrice ou MALDI consistant à cristalliser un composé tel que défini précédemment ou le composé 3-(5-(5-(méthylthio)thiophén-2-yl)thiophén-2-ylthio)propanenitrile. Ledit dispositif MALDI peut être un spectromètre de masse couplant une source d'ionisation laser assistée par une matrice et un analyseur à temps de vol.

Selon un mode de réalisation, ledit procédé est caractérisé en ce que ledit composé tel que défini précédemment, cristallisé, ou ledit composé de 3-(5-(5-(méthylthio)thiophén-2-yl)thiophén-2-ylthio)propanenitrile, cristallisé, est ensuite éventuellement dissout dans un solvant organique ou de l'eau comprenant un échantillon à analyser ou directement dissout dans l'échantillon à analyser, ledit solvant ou l'eau étant ensuite, le cas échéant, vaporisé formant au final une matrice cristallisée tel que défini précédemment ou de composé de 3-(5-(5-(méthylthio)thiophén-2-yl)thiophén-2-ylthio)propanenitrile comprenant l'échantillon à analyser.

L'échantillon peut être choisi parmi les extraits organiques bruts et les liquides biologiques.

Ce procédé peut avoir lieu avec un rapport molaire (molécules à analyser)/(molécules de matrice) compris entre 1/30 et 1/50, de préférence 1/39.

Le MT3P (**1**) présente les caractéristiques suivantes et les confère à la matrice qu'elle forme ou dans la composition dans laquelle elle entre :
- Le MT3P (**1**) nécessite une faible intensité d'irradiation du laser (5 à 10 %) pour le processus de désorption/ionisation, entraînant peu de fragmentations de l'analyte et de la matrice et l'obtention de spectres de masses MALDI-TOF caractérisés par des rapports signal sur bruit (S/N) élevés ;
- Le MT3P (**1**) présente une très haute sélectivité, vis à vis en particulier des alcaloïdes, hétérocycles azotés généralement doués d'activités pharmacologiques marquées. L'utilisation du MT3P (1) ne conduit ainsi à aucun résultat probant dans le cas de dérivés tels que stéroïdes (prégnolone, digitoxine), coumarines (imperatorine, E-notopterol), polyphénols (rutine, amenthoflavone), caroténoïdes (tocotriénols), peptides (angiotensine Il ou glycérides (1,3-diméthylglycérol, glycérol-1,3-distéarate)).

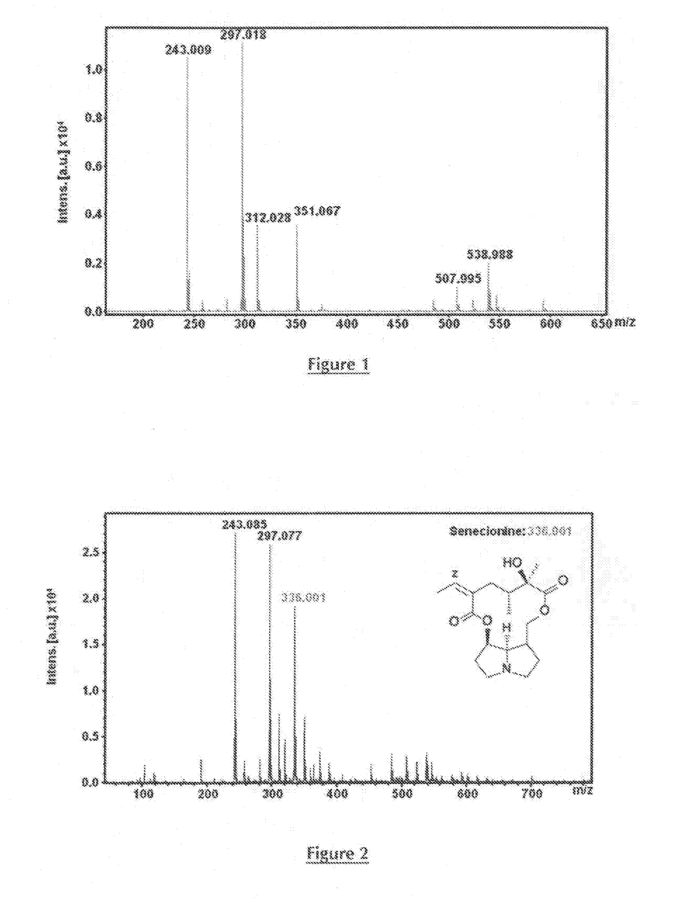

*A contrario* les alcaloïdes, quelque soit leur squelette de base (indoles, (iso)quinoléines, quinolizidines, pyrrolizidines, tropanes...) sont, dans la très grande majorité des cas, aisément détectés dans les mêmes conditions, tel qu'illustré dans le **tableau 2** ci-après. Les spectres obtenus présentent alors, au niveau des ions quasimoléculaires, des S/N équivalents ou supérieurs à ceux obtenus dans le cas de l'usage de matrices classiques telles l'acide 2-cyano-3-(4-hydroxyphenyl)propanoique (HCCA), le ditranol ou l'acide 2,5-dihydroxybenzoique (DHB). Cette excellente sensibilité de détection alliée à une grande spécificité vis à vis des analytes d'intérêt autorise ainsi, par exemple, la caractérisation directe, à partir d'un extrait organique brut de l'éthanol acide de *Senecio vulgaris,* de la sénécionine, dérivé de la rétronicine mutagène, tératogène et inducteur de tumeurs hépatiques tel que représenté sur le spectre de la **figure 2**. La présence de sénécionine doit être recherchée dans divers ingrédients alimentaires tels que l'huile de vipérine, *Echium plantagineum* [règlement (CE) n° 258/97 du Parlement Européen et du Conseil du 27 janvier 1997 ; Scientific Document: Opinion of the Panel on contaminants in the food chain [CONTAM] related to pyrrolizidine alkaloids as undesirable substances in animal feed, The EFSA Journal, 447, 1-51 (2007)].

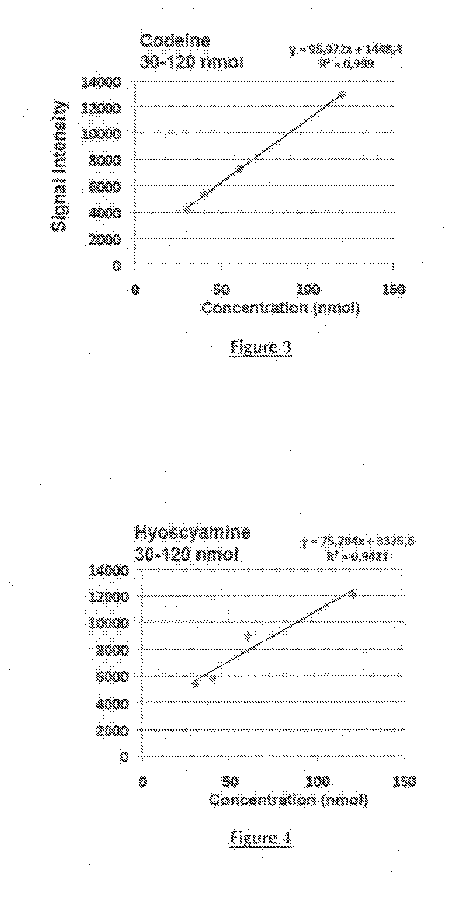

De très nombreuses analyses (semi)quantitatives sont ainsi réalisables avec une matrice de ou comprenant le MT3P (1). Selon la qualité des abaques obtenues, il est en effet possible de développer des méthodes de dosages spécifiques des alcaloïdes exploitant la sensibilité très élevée des spectromètres de masse de type MALDI-TOF conduisant à des limites de détection (LOD) ou de quantification (LOQ) très basses telles que représentées sur les **figures 3** et **4**. C'est le cas de la codéine (2), opioïde antalgique et antitussif ou bien de l'hyoscyamine (3), alcaloïde mydriatique représentés sur le **schéma 1** ci-après.

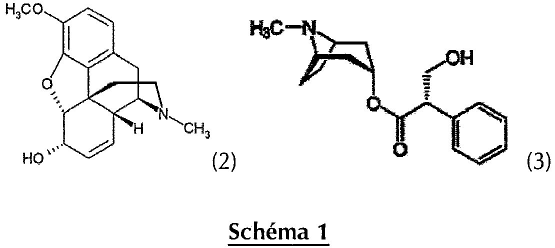

La matrice de 3-(5-(5-(méthylthio)thiophén-2-yl)thiophén-2-ylthio)propanenitrile selon l'invention a, en outre, l'avantage de pouvoir être préparée en 3 étapes simples avec un bon rendement de l'ordre de 80 % en poids à partir de produits commerciaux et donc pour un coût modique.

Elle présente, en outre, une sélectivité unique vis à vis des alcaloïdes ce qui autorise leur caractérisation et leur (semi)quantification par MALDI-TOF-MS à partir de mélanges complexes, sans préparations particulières des échantillons et avec d'excellents paramètres de limite de détection ou LOD et de limite de quantification ou LOQ.

Le MT3P est constituée d'un chromophore organisé autour d'un système π-conjugué plan absorbant à la longueur d'onde (337 nm) des lasers N₂ les plus souvent utilisés en SM MALDI-TOF d'une part et pourvue, via un espaceur, d'une fonction nitrile, d'autre part, susceptible de développer des interactions dipolaires avec les fonctions amines ou imines des composés recherchés. Il convient finalement de noter que l'emploi du MT3P, ne requiert que de faibles puissances d'irradiation laser et favorise la protonation des analytes.

Il faut également souligner que les protocoles de caractérisation ou de dosage des alcaloïdes ainsi élaborés ne nécessitent aucune modification des spectromètres MALDI-TOF dont la plupart des plateformes analytiques sont aujourd'hui équipées.

Dans le cadre des nouvelles réglementations REACH, l'utilisation de cette matrice peut représenter ainsi une aide précieuse au diagnostic lors des études de pharmacovigilance ou de toxicologie.

D'autres avantages tels que l'exploitation du MT3P en imagerie MALDI, pourront encore apparaître à l'homme du métier à la lecture des exemples ci-dessous, illustrés par les figures annexées, donnés à titre illustratif.

### EXEMPLES

### Exemple 1 : Préparation de la matrice MT3P (1)

Le composé 3-(5-(5-(méthylthio)thiophén-2-yl)thiophén-2-ylthio)propanenitrile (**1**) ou MT3P (**1**) ci-après est préparé à partir du 2-bromothiophène, en 3 étapes et avec un rendement global de 80 % tel que représenté selon le **Schéma 2** ci-après. Les conditions réactionnelles de cette synthèse sont:
- pour l'étape i) Magnésium, NidpppCl₂, Et₂O, reflux ;
- pour l'étape ii) nBuLi, Soufre et 2-bromopropionitrile, THF, température ambiante ;
- pour l'étape iii) Hydroxyde de césium et iodométhane, DMF/MeOH, température ambiante.

Le détail de ces étapes de cette synthèse est décrit dans le document « Mass Spectrom. », 2006; 41: 830-833.

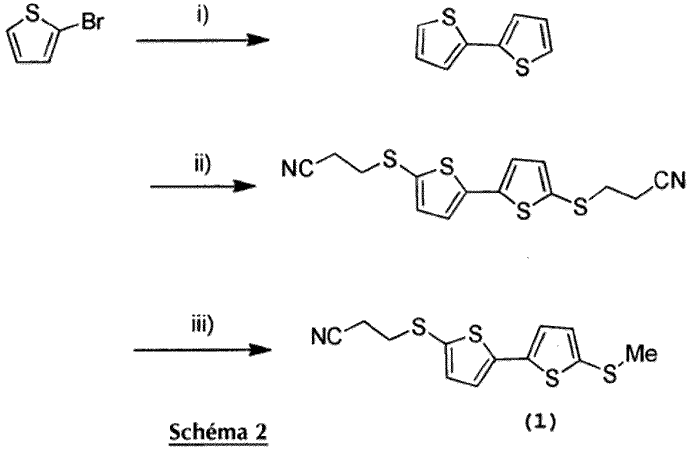

### Exemple 2 : Préparation de l'échantillon à analyser

On solubilise l'analyte dans un solvant organique adapté (e.g. CH₂Cl₂, MeOH, ...) à une concentration de 2,57 mmol/L. La solution préparée est stockée à une température de - 20°C. Avant chaque expérience réalisée en MALDI/TOF (Préparation de la matrice co-cristallisée avec l'échantillon comme expliqué dans la paragraphe suivant), cette solution est portée à température ambiante puis diluée au 1:3 dans MeOH.

### Exemple 3: Préparation de la matrice co-cristallisée avec l'échantillon à analyser

On réalise la préparation de la matrice co-cristallisée destinée à être utilisée dans un dispositif de spectrométrie de masse à désorption/ionisation sous laser assistée par matrice ou MALDI pour un seul analyte de la façon suivante : Un équivalent de solution d'analyte à une concentration de 0,861 mmol est mélangé avec deux équivalents de solution de matrice à une concentration de 33,67 mmol. Les concentrations finales sont 0,287 mmol/L pour l'analyte et 11.22 mmol/L pour la matrice. Pour un extrait brut, on peut, par exemple, mélanger un équivalent d'extrait brut (22 mg/mL) avec deux équivalents de matrice concentrée (30 mg/mL = 0,1 mmol/mL).

### Exemple 4: Tests de caractérisation d'alcaloïdes par SM MALDI-TOF avec le MT3P (1) pour matrice.

### Méthode de calibration du spectromètre :

La calibration du spectromètre est réalisée avec 20 microlitres d'un mélange de PEG400/MeOH dans des proportions en volume de 1/3. Cette solution est mélangée avec 20 microlitres de Ditranol à une concentration de 1010 mg/mL). 0.7 microlitres de cette solution sont déposés sur une coupelle MALDI. L'échantillon est irradié par un laser à un niveau d'énergie entre 35-40%. Les signaux du PEG-Na observés sont utilisés pour la calibration automatisée. On observe une concordance entre les signaux observés et les données de masse de PEG-Na en haute résolution.

Les alcaloides testés sont regroupés dans le **tableau 2** ci-dessous.

### Paramètres d'analyse du spectromètre :

### Les paramètres expérimentaux du MALDI-TOF-MS:

Source d'ion 1 : 19.00 kV,
Source d'ion 2 : 17.35 kV,
Lentilles : 9.60 kV,
Pulse pour l'extraction des ions : 200 ns,
Fréquence du laser : 5 ns,
Offset en tension du gain du détecteur : 1300 V,
Gain électronique : 100 mV,
Fenêtre d'acquisition : 20-2000 Da

**TABLEAU 2**

| **Alcatoïde** | **Formule** | **Masse monoisotopique** | **Détection** |
|---|---|---|---|
| 12-Déméthylthalrugosidine | C₃₇H₄₀N₂O₇ | 624,28 | + |
| Aconitine | C₃₄H₄₇NO₁₁ | 645,31 | ++ |
| Atropine | C₁₇H₂₃NO₃ | 289,17 | ++ |
| Berbérine | C2oHl8N04 | 336,12 | +++ |
| Boldine | C₁₉H₂₁NO₄ | 327,14 | ++ |
| Cholchicine | C₂₂H₂₅NO₆ | 399,17 | ++ |
| Clavuline | C₁₈H₁₉NO₄ | 313,13 | +++ |
| Codéine | C₁₈H₂₁NO₃ | 299,15 | ++ |
| Émétine | C₂₉H₄₀N₂O₄ | 480,30 | +++ |
| Fumaritine | C₂₀H₂₁NO₅ | 355,14 | +++ |
| Harmine | C₁₃H₁₂N₂O | 212,09 | +++ |
| L-Hyoscyamine | C₁₇H₂₃NO₃ | 289,17 | ++ |
| Limogine | C₂₀H₁₇NO₅ | 351,11 | +++ |
| Morphine | C₁₇H₁₉NO₃ | 285,14 | ++ |
| Nicotine | C₁₀H₁₄N₂ | 162.12 | - |
| Pilocarpine | C₁₁H₁₆N₂O₂ | 208,13 | ++ |
| Quinidine | C₂₀H₂₄N₂O₂ | 324,18 | +++ |
| Sénécionine | C₁₈H₂₅NO₅ | 335,17 | ++ |
| Spartéine | C₁₅H₂₆N₂ | 234,21 | +++ |
| Strychnine | C₂₁H₂₂N₂O₂ | 334,16 | ++ |
| Stylopine | C₁₉H₁₇NO₄ | 323,12 | +++ |
| Thalfoétidine | C₃₈H₄₂N₂O₇ | 638,30 | + |
| Thalibérine | C₃₇H₄₀N₂O₆ | 608,29 | +++ |
| Thaliglucinone | C₂₁H₁₉NO₅ | 365,13 | - |
| Yohimbine | C₂₁H₂₆N₂O₃ | 354,19 | ++ |

### COMPARATIFS ET EXEMPLES 5 à 16 : Tests de caractérisation de composés par SM MALDI-TOF avec différentes matrices, conformes ou non conformes à l'invention.

Pour chaque comparatif ou exemple, la préparation de l'échantillon à analyser et la préparation de la matrice co-cristallisée avec l'échantillon à analyser se fait de la même manière que celle exposée en exemple 2 et exemple 3 ci-dessus.

En outre, la méthode de calibration du spectromètre et les paramètres d'analyse du spectromètre sont identiques à ceux de l'exemple 4 ci-dessus.

Les mêmes alcaloïdes testés sur matrice MT3P dans l'exemple 4 sont testés sur des matrices différentes de MT3P, conformes ou non conformes à l'invention. Les résultats de détection des comparatifs, non conformes à l'invention, abrégés Cp6, Cp7, Cp14, Cp15 et Cp16, et les résultats de détections des exemples, conformes à l'invention, abrégés Ex4, Ex5, Ex8, Ex9, Ex10, Ex11, Ex12 et Ex13, sont regroupés dans le **tableau 3A** ci-dessous.

En outre, des molécules autres que des alcaloïdes sont testées également sur certaines de ces matrices différentes de MT3P. Les résultats de détection des comparatifs 6 et 7 (abrégés Cp6 à Cp7) et des exemples 5 et 8 à 13 (abrégés Ex5 et Ex8 à Ex13) sont regroupés dans le **tableau 3B** ci-dessous.

Les rapports signal/bruit des pics correspondant aux ions quasi-moléculaires sont classés en : élevé : + + + ; moyen : + + ; faible : + ; très faible : - et nul : 0. (nt signifiant non testé)

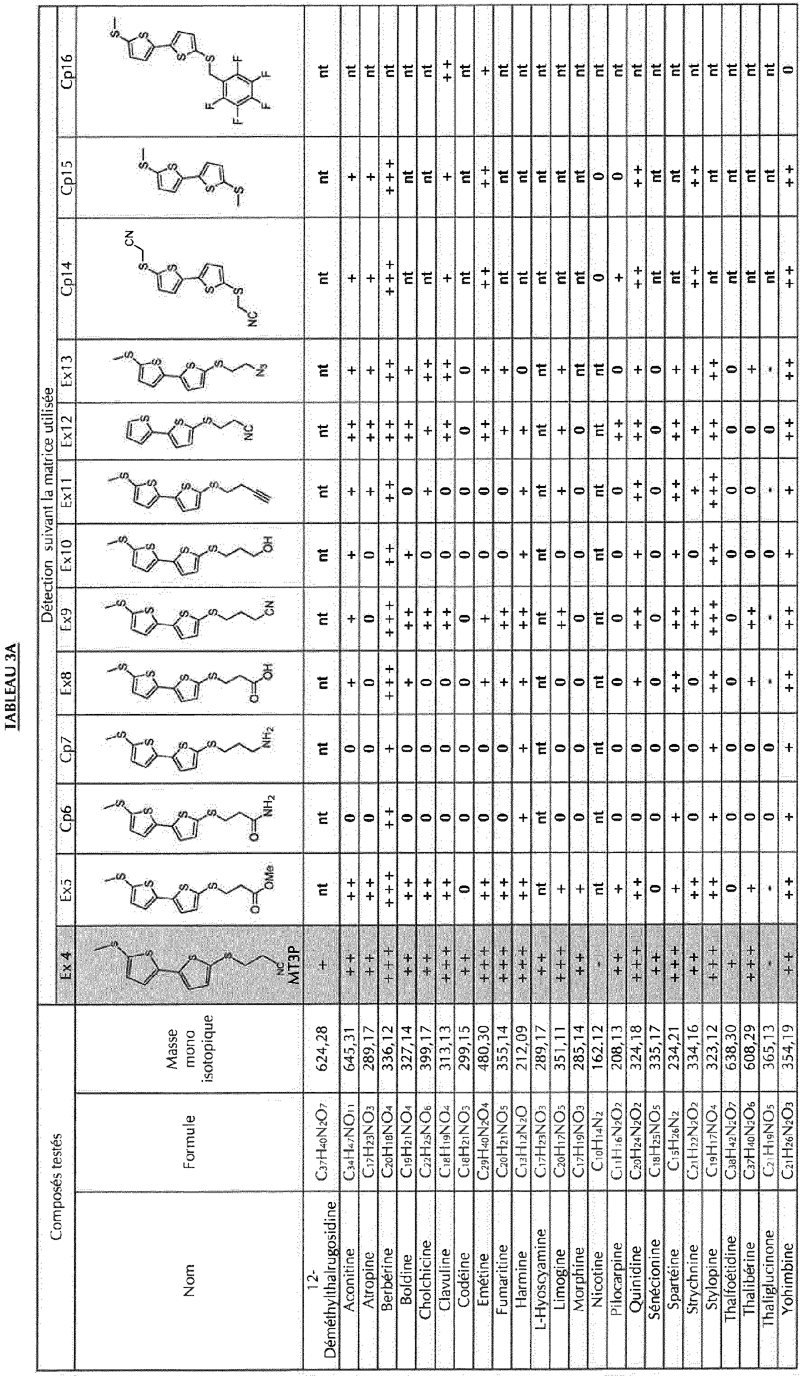

**Tableau 3B**

| Composés testés | Détection suivant la matrice utilisée | | | | | | | | |
|---|---|---|---|---|---|---|---|---|---|
| | Ex5 | Cp6 | Cp7 | Ex8 | Ex9 | Ex10 | Ex11 | Ex12 | Ex13 |
| | 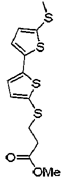 | 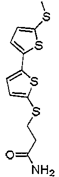 | 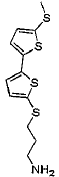 | 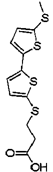 | 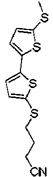 | 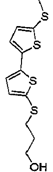 | 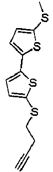 | 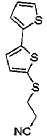 | 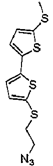 |
| 1.3-Dipalmitoylglycérol | 0 | 0 | 0 | 0 | 0 | 0 | 0 | 0 | 0 |
| Acide acétylsalicylique | 0 | 0 | 0 | 0 | 0 | 0 | 0 | 0 | 0 |
| Acide caféique | - | 0 | - | 0 | 0 | 0 | 0 | 0 | 0 |
| Acide chlorogénique | 0 | 0 | 0 | 0 | 0 | 0 | 0 | 0 | 0 |
| Acide fumarique | 0 | - | - | 0 | 0 | 0 | 0 | 0 | 0 |
| Amentoflavone | 0 | 0 | 0 | 0 | 0 | 0 | nt | 0 | 0 |
| Angiotensine II | 0 | 0 | 0 | 0 | 0 | 0 | 0 | 0 | 0 |
| Bergaptène | 0 | 0 | 0 | 0 | 0 | 0 | 0 | 0 | 0 |
| Caryophyllène | 0 | 0 | 0 | 0 | 0 | 0 | 0 | 0 | 0 |
| Coumarine | 0 | 0 | 0 | 0 | 0 | 0 | 0 | 0 | 0 |
| Curcumine | 0 | 0 | 0 | 0 | 0 | 0 | 0 | 0 | nt |
| Digitoxine | 0 | 0 | 0 | 0 | 0 | 0 | 0 | 0 | 0 |
| E-Notoptérol | 0 | 0 | 0 | 0 | 0 | 0 | 0 | 0 | 0 |
| Hespéridine | 0 | 0 | 0 | 0 | 0 | 0 | 0 | 0 | 0 |
| Isoimpératorine | 0 | 0 | 0 | 0 | 0 | 0 | 0 | 0 | 0 |
| Khelline | 0 | 0 | 0 | 0 | 0 | 0 | 0 | 0 | 0 |
| Pentaméthoxyflavone | - | 0 | 0 | 0 | 0 | 0 | - | - | - |
| Pregnolone | nt | nt | 0 | nt | 0 | 0 | 0 | 0 | 0 |
| Quercétine | 0 | 0 | 0 | 0 | 0 | 0 | 0 | 0 | 0 |
| Rutine | 0 | 0 | 0 | 0 | 0 | 0 | 0 | nt | 0 |
| Sitostérol | 0 | 0 | 0 | 0 | 0 | 0 | 0 | 0 | 0 |

### Listes des références

U.S. No. 6.104.028 ;
« The Scientist » 13 [12] : 18, le 7 juin 1999 ;
« Biophotonics International», juin 2001, 42-47.
CN 101644694;
« Journal of mass spectrometry », 2007 ; 42 :58-69 ;
« Analytica Chimica Acta, 649 (2009), 230-235 ;
« Analytical Chemistry », 1998, 70, 4376-4379 ;
« Journal of Mass Spectrometry », 2006, 41 : 830-833.

## Revendications

1. Utilisation du composé de formule (I) : 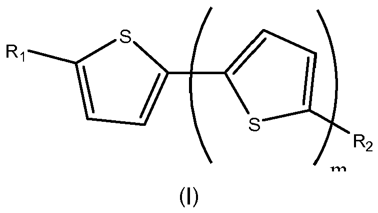 dans laquelle **m** est égal à 1, le groupement **R1** est choisi parmi les groupements -S(CH₂)ₙ-Y avec **n** un nombre entier choisi parmi 2 et 3 et **Y** un groupe fonctionnel choisi parmi-CN, -CO₂R3 et -OH, avec **R3** un atome d'hydrogène ou un groupement méthyle, et
dans laquelle le groupement **R2** est choisi parmi l'atome d'hydrogène et le groupement -SCH₃, comme matrice dans un dispositif de spectrométrie de masse à désorption/ionisation sous laser, assistée par une matrice ou MALDI.

2. Utilisation selon la revendication 1, dans laquelle :
• m = 1, R1 est un groupement -S(CH₂)₂CN et R2 un groupe -SCH₃ ;
• m = 1, R1 est un groupement -S(CH₂)₂CO₂CH₃ et R2 est un groupe - SCH₃ ;
• m = 1, R1 est un groupement -S(CH₂)₃CN et R2 est un groupe -SCH₃ ;
• m=1, R1 est un groupement -S(CH₂)₂CN et R2 est un atome d'hydrogène ;
• m = 1, R1 est un groupement -S(CH₂)₂CO₂H et R2 est un groupe -SCH₃ ; ou
• m=1, R1 est un groupement -S(CH₂)₂-CH₂OH et R2 est un groupe - SCH₃.

3. Utilisation selon l'une des revendications 1 à 3 dans laquelle le composé de formule (I) est le 3-(5-(5-(méthylthio)thiophén-2-yl)thiophén-2-ylthio)propanenitrile.

4. Utilisation selon l'une quelconque des revendications 1 à 3 dans laquelle ledit dispositif MALDI est couplé à un analyseur à temps de vol.

5. Utilisation selon l'une quelconque des revendications 1 à 4, pour analyser qualitativement et quantitativement un mélange de molécules ou échantillon comprenant au moins une molécule de masse < 800 Da.

6. Utilisation selon la revendication 5, dans lequel ladite molécule de masse < 800 Da est choisie parmi les alcaloïdes.

7. Utilisation selon la revendication 6, dans laquelle les alcaloïdes sont choisis parmi :
• Le groupe des Azolidines ;
• Le groupe des Azines ;
• Le groupe des Tropanes ;
• Le groupe des Quinoléines ;
• Le groupe des Isoquinolines ;
• Le groupe des Indoles ;
• Le groupe des Terpénoides ;
• Le groupe des Bétaines ;
• Le groupe des Pyrazoles.

8. Utilisation selon la revendication 6, dans laquelle les alcaloïdes sont choisis parmi (i) l'Aniracetam, la Anisomycin, le CX614, le Dextromoramide, le Diphenyl prolinol, l'Acide Domoique, la Histapyrrodine, l'Acide Kainique, la Methdilazine, l'Oxaceprol, le Prolintane, la Pyrrobutamine, l'Hygrine, la Cuscohygrine ; (ii) la Pipéridine, la Conicine, la Trigonelline, l'Arecaidine, la Guvacine, la Cytisine, la Pelletierine ; (iii), la Cocaïne, l'Ecgonine, la Scopolamine ; (iv) l'Acridine, l'Acide bicinchoninique, la Broxyquinoline, le Chlorquinaldol, le Cinchophen, le Clioquinol, le Dequalinium, la Dihydroquinine, la Dihydroquinidine, l'Hydroxychloroquine, la 8-Hydroxyquinoline, 1'lodoquinol, l'Acide Kynurenique, la Mefloquine, la Nitroxoline, l'Oxycinchophen, la Primaquine, la Quinine, le TSQ, le Topotecan, l'Acide Xanthurenique, la Brucine, la Veratrine, la Cevadine, l'Echinopsine, les Aminoquinoléines tels que la Chloroquine, l'Hydroxychloroquine, la Primaquine, les 8-Aminoquinoléines tels que la Tafenoquine, la Rhodoquine, la Pamaquine; (v) la Dimethisoquine, le Quinapril, le Quinapirilat, le Debrisoquine, le 2,2'-Hexadecaméthylènediisoquinolinium dichlorure, le N-Laurylisoquinolinium bromure, le Narcéine, l'Hydrastine, les alcaloides de l'opium naturels tels que la Thébaine, la Papavérine, la Narcotine, la Noscapine, les alcaloides de l'opium semi-synthétiques tels que l'Hydromorphone, l'Hydrocodone, l'Héroïne, les alcaloïdes de l'opium synthétiques tels que le Fentanyl, la Pethidine, la Methadone, le Propoxyphène ; (vi) les Ergolines tels que les alcaloides de l'ergot de seigle tels que l'Ergométrine, l'Ergotamine, l'Ergosine, l'Ergovaline, l'Ergokryptine, l'Ergocornine, l'Ergocristine, l'Acide lysergique, l'Ergine, le LSD, (vii) les Bêta-carbolines tels que la Réserpine, l'Emétine ; (viii) les alcaloides de l'aconit napel tel que la Solanidine, (ix) la Solasodine, la Batrachotoxine, la Delphinine, (x) les stéroides tels que la Solanine, la Samandarin ; (xi) la Muscarine, la Choline, la Neurine.

9. Utilisation selon la revendication 6, dans laquelle ladite molécule de masse < 800 Da est choisie parmi 12-Déméthylthalrugosidine, Aconitine, Atropine, Berbérine, Boldine, Cholchicine, Clavuline, Codéine, Émétine, Fumaritine, Harmine, L-Hyoscyamine, Limogine, Morphine, Nicotine, Pilocarpine, Quinidine, Sénécionine, Spartéine, Strychnine, Stylopine, Thalfoétidine, Thalibérine, Thaliglucinone et Yohimbine.

10. Utilisation selon l'une des revendications 5 à 6, dans laquelle ledit mélange ou échantillon est un mélange choisi parmi des extraits organiques bruts, ou toute fraction obtenue à partir desdits extraits organiques bruts, et des liquides biologiques, d'origine humaine ou animale.

11. Utilisation selon la revendication 10 dans laquelle ledit échantillon est choisi parmi les phanères, le sang et le plasma, les liquides des muqueuses génitales, les liquides de la peau, les liquides d'épanchement et des cavités closes, les liquides du système digestif et urinaire.

12. Composé de formule (I) : 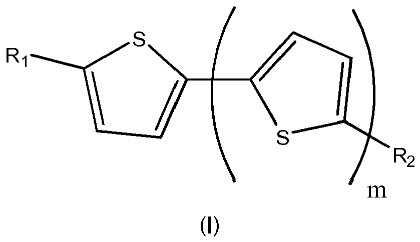 dans laquelle m est égal à 1, le groupement R1 est choisi parmi les groupements -S(CH₂)ₙ-Y avec n un nombre entier choisi parmi 2 et 3 et Y un groupe fonctionnel choisi parmi-CN, -CO₂R3 et -OH, avec R3 un atome d'hydrogène ou un groupement méthyle, et
dans laquelle le groupement R2 est le groupement -SCH₃,
ou dans laquelle m = 1, R1 est un groupement -S(CH₂)₂CN et R2 est un atome d'hydrogène ;
à l'exclusion du 3-(5-(5-(méthylthio)thiophén-2-yl)thiophén-2-ylthio)propanenitrile.

13. Composé selon la revendication 12, où :
• m=1, R1 est un groupement -S(CH₂)₂CO₂CH₃ et R2 est un groupe - SCH₃ ;
• m=1, R1 est un groupement -S(CH₂)₃CN et R2 est un groupe -SCH₃ ;
• m=1, R1 est un groupement -S(CH₂)₂CO₂H et R2 est un groupe -SCH₃ ; ou
• m=1, R1 est un groupement -S(CH₂)₂-CH₂OH et R2 est un groupe - SCH₃.

14. Utilisation d'un composé selon la revendication 12 ou 13 ou de 3-(5-(5-(méthylthio)thiophén-2-yl)thiophén-2-ylthio)propanenitrile pour fabriquer une matrice destinée à être utilisée dans un dispositif de spectrométrie de masse à désorption/ionisation sous laser assistée par matrice ou MALDI.

15. Procédé de fabrication d'une matrice destinée à être utilisée dans un dispositif de spectrométrie de masse à désorption/ionisation sous laser assistée par matrice ou MALDI consistant à cristalliser un composé selon la revendication 12 ou 13 ou le composé 3-(5-(5-(méthylthio)thiophén-2-yl)thiophén-2-ylthio)propanenitrile.

16. Procédé selon la revendication 15 dans lequel ledit dispositif MALDI est un spectromètre de masse couplant une source d'ionisation laser assistée par une matrice et un analyseur à temps de vol.

17. Procédé selon la revendication 14 ou 15, **caractérisé en ce que** ledit composé, selon la revendication 12 ou 13, cristallisé ou ledit composé de 3-(5-(5-(méthylthio)thiophén-2-yl)thiophén-2-ylthio)propanenitrile cristallisé est ensuite dissout dans un solvant organique ou de l'eau comprenant un échantillon à analyser, ledit solvant ou l'eau étant ensuite vaporisé formant au final une matrice cristallisée de composé selon l'une quelconque des revendications 13 à 15 ou de composé de 3-(5-(5-(méthylthio)thiophén-2-yl)thiophén-2-ylthio)propanenitrile comprenant l'échantillon à analyser.

18. Procédé selon la revendication 17, dans lequel l'échantillon est choisi parmi les extraits organiques bruts ou toute fraction obtenue à partir d'extraits organiques bruts et les liquides biologiques, d'origine humaine ou animale.

19. Procédé selon l'une quelconque des revendications 17 ou 18, dans lequel le rapport molaire (molécules à analyser)/(molécules de matrice) est compris entre 1/30 et 1/50.

20. Procédé de caractérisation et/ou de quantification de molécules, ayant une masse < 800 Da, présentes dans un échantillon par spectrométrie de masse à temps de vol à désorption/ionisation sous laser assistée par matrice comprenant une étape de :
> Fabrication d'une matrice avec un composé selon l'une des revendications 13 à 15 ou avec un composé de 3-(5-(5-(méthylthio)thiophén-2-yl)thiophén-2-ylthio)propanenitrile ;
> Mélange dudit échantillon avec ladite matrice, éventuellement en présence d'un solvant organique ou d'eau ;
> Vaporisation du solvant ou de l'eau, le cas échéant ;
> Co-cristallisation de ladite matrice et desdites molécules et formation d'un cristal de matrice comprenant lesdites molécules réparties dans tout ledit cristal ;
> Ionisation par un faisceau laser de ladite matrice co-cristallisée avec l'échantillon, obtenue précédemment ;
➢ Établissement d'un spectrogramme.

21. Procédé selon la revendication 20, **caractérisé en ce que** l'échantillon est choisi parmi des extraits organiques bruts, ou toute fraction obtenue à partir desdits extraits organiques bruts, et des liquides ou des échantillons biologiques, d'origine humaine ou animale.

22. Procédé selon la revendication 20 ou 21, **caractérisé en ce que** l'échantillon est choisi parmi le sang, le plasma, les liquides des muqueuses génitales, les liquides de la peau, les liquides d'épanchement et des cavités closes, les liquides du système digestif et urinaire et les phanères.

## Patentansprüche

1. Verwendung der Verbindung mit der Folgenden Formel (I): 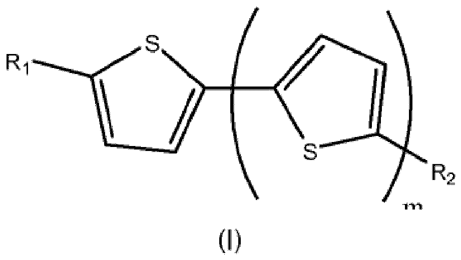 bei der m gleich 1 ist, die Gruppe R1 ausgewählt ist aus den Gruppen -S(CH₂)ₙ-Y, wobei n eine ganze Zahl, ausgewählt aus 2 und 3, ist, und Y eine funktionelle Gruppe, ausgewählt aus -CN, _CO₂R₃ und -OH ist, wobei R3 ein Wasserstoffatom oder eine Methylgruppe ist, und
wobei die Gruppe R2 ausgewählt ist aus dem Wasserstoffatom und der Gruppe -SCH₃ als Matrix in einer Massenspektrometrievorrichtung mit Matrix-unterstützter Laser-Desorption/Ionisation oder MALDI.

2. Verwendung nach Anspruch 1, wobei
- m=1, R1 eine Gruppe -S(CH₂)₂CN und R2 eine Gruppe -SCH₃ ist;
- m=1, R1 eine Gruppe -S(CH₂)₂CO₂CH₃ und R2 eine Gruppe -SCH₃ ist;
- m=1, R1 eine Gruppe -S(CH₂)₃CN und R2 eine Gruppe -SCH₃ ist;
- m=1, R1 eine Gruppe -S(CH₂)₂CN und R2 ein Wasserstoffatom ist;
- m=1, R1 eine Gruppe -S(CH₂)₂CO₂H und R₂ eine Gruppe -SCH₃ ist; oder
- m=1, R1 eine Gruppe -S (CH₂) ₂-CH₂OH und R₂ eine Gruppe -SCH₃ ist.

3. Verwendung nach einem der Ansprüche 1 bis 3, wobei die Verbindung mit der Formel (I) das 3-(5-(5-(Methylthio)thiophen-2-yl)thiophen-2-ylthio)propannitril ist.

4. Verwendung nach einem der Ansprüche 1 bis 3, wobei die MALDI-Vorrichtung mit einer Flugzeitanalysevorrichtung gekoppelt ist.

5. Verwendung nach einem der Ansprüche 1 bis 4, um qualitativ und quantitativ eine Mischung aus Molekülen oder eine Probe zu analysieren, die mindestens ein Molekül mit der Masse < 800 Da umfasst.

6. Verwendung nach Anspruch 5, wobei das Molekül mit der Masse < 800 Da aus den Alkaloiden ausgewählt ist.

7. Verwendung nach Anspruch 6, wobei die Alkaloide ausgewählt sind aus:
- der Gruppe der Azolidine;
- der Gruppe der Azine;
- der Gruppe der Tropane;
- der Gruppe der Chinoline;
- der Gruppe der Isochinoline;
- der Gruppe der Indole;
- der Gruppe der Terpenoide;
- der Gruppe der Betaine;
- der Gruppe der Pyrazole.

8. Verwendung nach Anspruch 6, wobei die Alkaloide ausgewählt sind aus (i) dem Aniracetam, dem Anisomycin, dem CX614, dem Dextromoramid, dem Diphenylprolinol, der Domoinsäure, dem Histapyrrodin, der Kainsäure, dem Methdilazin, dem Oxaceprol, dem Prolintan, dem Pyrrobutamin, dem Hygrin, dem Cuscohygrin; (ii) dem Piperidin, dem Conicin, dem Trigonellin, dem Arecaidin, dem Guvacin, dem Cytisin, dem Pelletierin; (iii) dem Kokain, dem Ecgonin, dem Scopolamin; (iv) dem Acridin, der Bicinchoninsäure, dem Broxyquinolin, dem Chlorquinaldol, dem Cinchophen, dem Clioquinol, dem Dequalinium, dem Dihydrochinin, dem Dihydrochinidin, dem Hydroxychloroquin, dem 8-Hydroxychinolin, dem Iodochinol, der Kynurensäure, dem Mefloquin, dem Nitroxolin, dem Oxycinchophen, dem Primaquin,dem Chinin, dem TSQ, dem Topotecan, der Xanthurensäure, dem Brucin, dem Veratrin dem Cevadin, dem Echinopsin, den Aminochinolinen wie z.B. dem Chloroquin, dem Hydroxychloroquin, dem Primaquin, den 8-Aminochinolinen wie z.B. dem Tafenoquin, dem Rhodoquin, dem Pamaquin; (v) dem Dimethisoquin, dem Quinapril, dem Quinapirilat, dem Debrisoquin, dem 2,2'-Hexadecamethylendiisochinolindichlorid, dem N-Laurylisochinolinbromid, dem Narcein, dem Hydrastin, den natürlichen Alkaloiden des Opiums wie z.B. dem Thebain, dem Papaverin, dem Narcotin, dem Noscapin, den halbsynthetischen Alcaloiden des Opiums wie z.B. dem Hydromorphon, dem Hydrocodon, dem Heroin, den synthetischen Alcaloiden des Opiums wie z.B. dem Fentanyl, dem Pethidin, dem Methadon, dem Propoxyphen; (vi) den Ergolinen wie z.B. den Alcaloiden des Mutterkorns wie z.B. dem Ergometrin, dem Ergotamin, dem Ergosin, dem Ergovalin, dem Ergokryptin, dem Ergocornin, dem Ergocristin, der Lysergsäure, dem Ergin, dem LSD, (vii) den Betacarbolinen wie z.B. dem Reserpin, dem Emetin; (viii) den Alcaloiden des blauen Eisenhuts wie z.B. dem Solanidin, (ix) dem Solasodin, dem Batrachotoxin, dem Delphinin, (x) den Steroiden wie z.B. dem Solanin, dem Samandarin; (xi) dem Muscarin, dem Cholin, dem Neurin.

9. Verwendung nach Anspruch 6, wobei das Molekül mit der Masse < 800 Da ausgewählt ist aus 12-Demethylthalrugosidin, Aconitin, Atropin, Berberin, Boldin, Cholchicin, Clavulin, Codein, Emetin, Fumaritin, Harmin, L-Hyoscyamin, Limogin, Morphin, Nicotin, Pilocarpin, Chinidin, Senecionin, Spartein, Strychnin, Stylopin, Thalfoetidin, Thaliberin, Thaliglucinon und Yohimbin.

10. Verwendung nach einem der Ansprüche 5 bis 6, wobei die Mischung oder die Probe eine Mischung ist, ausgewählt aus rohen organischen Extrakten oder jeder Fraktion, die auf der Grundlage von rohen organischen Extrakten erhalten wird, und biologischen Flüssigkeiten menschlichen oder tierischen Ursprungs.

11. Verwendung nach Anspruch 10, wobei die Probe ausgwählt ist aus den Phaneren, dem Blut und dem Plasma, den Flüssigkeiten der genitalen Schleimhäute, den Flüssigkeiten der Haut, den Flüssigkeiten von Ergüssen und geschlossenen Hohlräumen, den Flüssigkeiten des Verdauungs- und Harnsystems.

12. Verbindung mit der folgenden Formel (I): 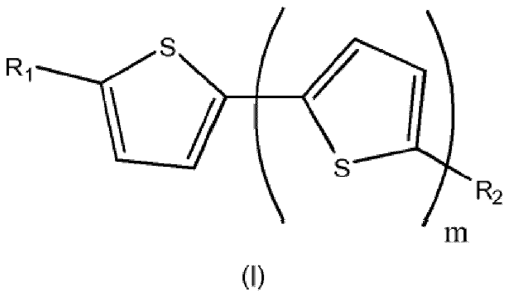 wobei m gleich 1 ist, die Gruppe R1 ausgewählt ist aus den Gruppen -S(CH₂)ₙ-Y wobei n eine ganze Zahl, ausgewählt aus 2 und 3, ist, und Y eine funktionelle Gruppe, ausgewählt aus -CN, -CO₂R3 und -OH ist, wobei R3 ein Wasserstoffatom oder eine Methylgruppe ist, und wobei die Gruppe R2 die Gruppe -SCH₃ ist, oder wobei m=1 ist, R1 eine Gruppe -S(CH₂)₂CN und R2 ein Wasserstoffatom ist;
mit Ausnahme des 3-(5-(5-(Methylthio)thiophen-2-yl)thiophen-2-ylthio)propannitril.

13. Verbindungen nach Anspruch 12, wobei
- m=1, R1 eine Gruppe S(CH₂)₂CO₂CH₃ und R2 eine Gruppe -SCH₃ ist;
- m=1, R1 eine Gruppe -S(CH₂)₃CN und R2 eine Gruppe -SCH₃ ist;
- m=1, R1 eine Gruppe -S(CH₂)₂CO₂H und R2 eine Gruppe -SCH₃ ist;
- m=1, R1 eine Gruppe -S(CH₂)₂-CH₂OH und R2 eine Gruppe -SCH₃ ist.

14. Verwendung einer Verbindung nach Anspruch 12 oder 13 oder von 3-(5-(5-(Methylthio)thiophen-2-yl)thiophen-2-ylthio)propannitril, um eine Matrix herzustellen, die ausgelegt ist, um in einer Massenspektrometrievorrichtung mit Matrix-unterstützter Laser-Desorption/Ionisation oder MALDI verwendet zu werden.

15. Verfahren zur Herstellung einer Matrix, die ausgelegt ist, um in einer Massenspektrometrievorrichtung mit Matrix-unterstützter Laser-Desorption/Ionisation oder MALDI verwendet zu werden, die darin besteht, eine Verbindung nach Anspruch 12 oder 13 oder die Verbindung 3-(5-(5-(Methylthio)thiophen-2-yl)thiophen-2-ylthio)propannitril zu kristallisieren.

16. Verfahren nach Anspruch 15, wobei die MALDI-Vorrichtung ein Massenspektrometer ist, der eine Matrix-unterstützte Laser-Ionisationsquelle und eine Flugzeitanalysevorrichtung miteinander koppelt.

17. Verfahren nach Anspruch 14 oder 15, **dadurch gekennzeichnet, dass** die kristallisierte Verbindung nach Anspruch 12 oder 13 oder die kristallisierte Verbindung von 3-(5-(5-(Methylthio)thiophen-2-yl)thiophen-2-ylthio)propannitril anschließend in einem organischen Lösemittel oder Wasser, umfassend eine zu analysierende Probe, gelöst wird, wobei das Lösemittel oder das Wasser anschließend verdampft wird und schließlich eine kristallisierte Matrix der Verbindung nach einem der Ansprüche 13 bis 15 oder der Verbindung von 3-(5-(5-(Methylthio)thiophen-2-yl)thiophen-2-ylthio)propannitril mit der zu analysierenden Probe bildet.

18. Verfahren nach Anspruch 17, wobei die Probe ausgewählt ist aus den rohen organischen Extrakten oder jeder Fraktion, die auf der Grundlage von rohen organischen Extrakten erhalten wird, und den biologischen Flüssigkeiten menschlichen oder tierischen Ursprungs.

19. Verfahren nach einem der Ansprüche 17 oder 18, wobei das Molverhältnis (zu analysierende Moleküle)/(Matrixmoleküle) zwischen 1/30 und 1/50 liegt.

20. Verfahren zur Charakterisierung und/oder Quantifizierung von Molekülen mit einer Masse < 800 Da, die in einer Probe vorhanden sind, durch Flugzeitmassenspektromerie mit Matrix-unterstützter Laser-Desorption/Ionisation, umfassend einen Schritt des:
- Herstellens einer Matrix mit einer Verbindung nach einem der Ansprüche 13 bis 15 oder mit einer Verbindung von 3-(5-(5-(Methylthio)thiophen-2-yl)thiophen-2-ylthio)propannitril;
- Mischens der Probe mit der Matrix, eventuell bei Anwesenheit eines organischen Lösemittels oder von Wasser:
- gegebenenfalls Verdampfens des Lösemittels oder des Wassers;
- Cokristallisierens der Matrix und der Moleküle und Bildens eines Matrixkristalls, umfassend die Moleküle, die im gesamten Kristall verteilt sind;
- Ionisierens durch ein Laserbündel der cokristallisierten Matrix mit der Probe, die zuvor erhalten wurde;
- Erstellens eines Spektrogramms.

21. Verfahren nach Anspruch 20, **dadurch gekennzeichnet, dass** die Probe ausgewählt ist aus rohen organischen Extrakten, oder jeder Fraktion, die auf der Grundlage der rohen organischen Extrakte erhalten wird, und biologischen Flüssigkeiten menschlichen oder tierischen Ursprungs.

22. Verfahren nach Anspruch 20 oder 21, **dadurch gekennzeichnet, dass** die Probe ausgewählt ist aus dem Blut, dem Plasma, den Flüssigkeiten der genitalen Schleimhäute, den Flüssigkeiten der Haut, den Flüssigkeiten von Ergüssen und geschlossenen Hohlräumen, den Flüssigkeiten des Verdauungs- und Harnsystems und den Phaneren.

## Claims

1. Use of the compound of formula (I): 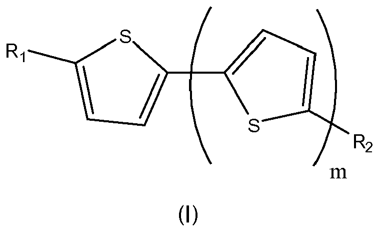 wherein **m** is 1, the group **R1** is selected from groups -S(CH₂)ₙ-Y where **n** is an integer selected from 2 and 3 and **Y** is a functional group selected from -CN,-CO₂R3, and -OH, with **R3** being a hydrogen atom or a methyl group, and wherein the group **R2** is selected from a hydrogen atom and a -SCH₃ group, as matrix in a matrix-assisted laser desorption/ionization, or MALDI, mass spectrometry device.

2. Use as claimed in claim 1, wherein:
• m = 1, R1 is a -S(CH₂)₂CN group, and R2 is a -SCH₃ group;
• m = 1, R1 is a -S(CH₂)₂CO₂CH₃ group, and R2 is a -SCH₃ group;
• m=1, R1 is a -S(CH₂)₃CN group, and R2 is a -SCH₃ group;
• m =1, R1 is a -S(CH₂)₂CN group, and R2 is hydrogen atom;
• m =1, R1 is a -S(CH₂)₂CO₂H group, and R2 is a -SCH₃ group; or
• m =1, R1 is a -S(CH₂)₂-CH₂0H group, and R2 is a -SCH₃ group.

3. Use as claimed in any of claims 1 to 2, wherein the compound of formula (I) is 3-(5-(5-(methylthio)thiophen-2-yl)thiophen-2-ylthio)propanenitrile.

4. Use as claimed in any one of claims 1 to 3, wherein said MALDI device is coupled to a time-of-flight analyzer.

5. Use as claimed in any one of claims 1 to 4, for qualitative and quantitative analysis of a mixture of molecules or sample comprising at least one molecule of mass < 800 Da.

6. Use as claimed in claim 5, wherein said molecule of mass < 800 Da is selected from alkaloids.

7. Use as claimed in claim 6, wherein the alkaloids are selected from:
• the group of the Azolidines;
• the group of the Azines;
• the group of the Tropanes;
• the group of the Quinoleines;
• the group of the Isoquinolines;
• the group of the Indoles;
• the group of the Terpenoids;
• the group of the Betaines;
• the group of the Pyrazoles.

8. Use as claimed in claim 6, wherein the alkaloids are selected from (i) Aniracetam, Anisomycin, CX614, Dextromoramide, Diphenylprolinol, Domoic acid, Histapyrrodine, Kainic acid, Methdilazine, Oxaceprol, Prolintane, Pyrrobutamine, Hygrine, Cuscohygrine; (ii) Piperidine, Conicine, Trigonelline, Arecaidine, Guvacine, Pilocarpine, Cytisine, Nicotine, Sparteine, Pelletierine; (iii) atropine, L-Hyoscyamine, Cocaine, Ecgonine, Scopolamine; (iv) Acridine, Bicinchoninic acid, Broxyquinoline, Chlorquinaldol, Cinchophen, Clioquinol, Dequalinium, Dihydroquinine, Dihydroquinidine, Hydroxychloroquine, 8-Hydroxyquinoline, lodoquinol, Kynurenic acid, Mefloquine, Nitroxoline, Oxycinchophen, Primaquine, Quinine, Quinidine, TSQ, Topotecan, Xanthurenic acid, Strychnine, Brucine, Veratrine, Cevadine, Echinopsine, Aminoquinolines such as Chloroquine, Hydroxychloroquine, Primaquine, 8-Aminoquinolines such as Tafenoquine, Rhodoquine, Pamaquine; (v) Dimethisoquine, Quinapril, Quinapirilat, Debrisoquine, 2,2'-Hexadecamethylenediisoquinolinium dichloride, N-Laurylisoquinolinium bromide, Narceine, Hydrastine, Berberine, natural opium alkaloids such as Morphine, Codeine, Thebaine, Papaverine, Narcotine, Noscapine, semisynthetic opium alkaloids such as Hydromorphone, Hydrocodone, Heroin, synthetic opium alkaloids such as Fentanyl, Pethidine, Methadone, Propoxyphene; (vi) Ergolines such as rye ergot alkaloids such as Ergometrine, Ergotamine, Ergosine, Ergovaline, Ergokryptine, Ergocornine, Ergocristine, Lysergic acid, Ergine, LSD, (vii) Beta-carbolines such as Harmine, Yohimbine, Reserpine, Emetine; (viii) aconite alkaloids such as Aconitine, Solanidine, (ix) Solasodine, Batrachotoxin, Delphinine, (x) steroids such as Solanine, Samandarin; (xi) Muscarine, Choline, Neurine.

9. Use as claimed in claim 6, wherein said molecule of mass < 800 Da is selected from 12-Demethylthalrugosidine, Aconitine, Atropine, Berberine, Boldine, Cholchicine, Clavuline, Codeine, Emetine, Fumaritine, Harmine, L-Hyoscyamine, Limogine, Morphine, Nicotine, Pilocarpine, Quinidine, Senecionine, Sparteine, Strychnine, Stylopine, Thalfoetidine, Thaliberine, Thaliglucinone, and Yohimbine.

10. Use as claimed in either of claims 5 and 6, wherein said mixture or sample is a mixture selected from crude organic extracts, or any fraction obtained from said crude organic extracts, and biological fluids, of human or animal origin.

11. Use as claimed in claim 10, wherein said sample is selected from epidermal derivatives, blood and plasma, fluids of the genital mucosae, fluids of the skin, effusive fluids and closed-cavity fluids, and fluids of the digestive and urinary system.

12. Compound of formula (I): 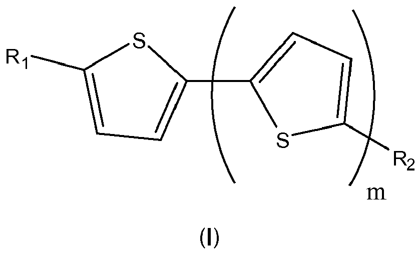 wherein m is 1, the group **R1** is selected from groups -S(CH₂)ₙ-Y where **n** is an integer selected from 2 and 3 and **Y** is a functional group selected from -CN,-CO₂R3, and -OH, with **R3** being a hydrogen atom or a methyl group, and wherein the group **R2** is a methyl group,
or wherein m=1, R1 is a -S(CH₂)₂CN group, and R2 is a hydrogen atom;
with the exception of 3-(5-(5-(methylthio)thiophen-2-yl)thiophen-2-ylthio)-propanenitrile.

13. Compound as claimed in claim 12, in which:
• m = 1, R1 is a -S(CH₂)₂CO₂CH₃ group, and R2 is a -SCH₃ group;
• m = 1, R1 is a -S(CH₂)₃CN group, and R2 is a -SCH₃ group;
• m = 1, R1 is a -S(CH₂)₂CO₂H group, and R2 is a -SCH₃ group; or
• m = 1, R1 is a -S(CH₂)₂-CH₂0H group, and R2 is a -SCH₃ group.

14. Use of a compound as claimed in claim 12 or 13 or of 3-(5-(5-(methylthio)thiophen-2-yl)thiophen-2-ylthio)propanenitrile for producing a matrix intended for use in a matrix-assisted laser desorption/ionization, or MALDI, mass spectrometry device.

15. Method for producing a matrix intended for use in a matrix-assisted laser desorption/ionization, or MALDI, mass spectrometry device, which comprises crystallizing a compound as claimed in claim 12 to 13 or the compound 3-(5-(5-(methylthio)thiophen-2-yl)thiophen-2-ylthio)propanenitrile.

16. Method as claimed in claim 15, wherein said MALDI device is a mass spectrometer coupling a matrix-assisted laser ionization source and a time-of-flight analyzer.

17. Method as claimed in claim 14 or 15, **characterized in that** said compound, as claimed in claim 12 or 13, that is crystallized, or said 3-(5-(5-(methylthio)thiophen-2-yl)thiophen-2-ylthio)propanenitrile compound that has been crystallized, is subsequently dissolved in an organic solvent or water comprising a sample for analysis, said solvent or water being subsequently vaporized to form eventually a crystallized matrix of compound as claimed in claims 13 to 15 or of 3-(5-(5-(methylthio)thiophen-2-yl)thiophen-2-ylthio)propanenitrile compound comprising the sample for analysis.

18. Method as claimed in claim 17, wherein the sample is selected from crude organic extracts, or any fraction obtained from crude organic extracts, and biological fluids, of human or animal origin.

19. Method as claimed in either of claims 17 or 18, wherein the molar (molecules for analysis)/(molecules of matrix) ratio is between 1/30 and 1/50.

20. Method for characterizing and/or quantifying molecules, having a mass < 800 Da, present in a sample, by matrix-assisted laser desorption/ionization time-of-flight mass spectrometry, comprising a step of:
> Producing a matrix with a compound as claimed in any of claims 13 to 15 or with a 3-(5-(5-(methylthio)thiophen-2-yl)thiophen-2-ylthio)-propanenitrile compound;
> Mixing said sample with said matrix, optionally in the presence of an organic solvent or water;
> Vaporizing the solvent or the water, as appropriate;
> Cocrystallizing said matrix and said molecules, and forming a matrix crystal comprising said molecules distributed throughout said crystal;
> Subjecting said matrix cocrystallized with the sample, obtained beforehand, to ionization by a laser beam;
> Establishing a spectrogram.

21. Method as claimed in claim 20, **characterized in that** the sample is selected from crude organic extracts, or any fraction obtained from said crude organic extracts, and biological fluids or samples, of human or animal origin.

22. Method as claimed in claim 20 or 21, **characterized in that** the sample is selected from blood, plasma, fluids of the genital mucosae, fluids of the skin, effusive and closed-cavity fluids, fluids of the digestive and urinary system, and epidermal derivatives.
